(19) Europäisches Patentamt
European Patent Office
Office européen des brevets

(11) Veröffentlichungsnummer: **0 093 963**
**A1**

(12) **EUROPÄISCHE PATENTANMELDUNG**

(21) Anmeldenummer: **83104152.0**

(22) Anmeldetag: **28.04.83**

(51) Int. Cl.³: **C 07 C 43/178**, C 07 C 43/196, C 07 C 41/03, C 07 C 41/22, A 01 N 31/00

(30) Priorität: **06.05.82 DE 3216894**

(43) Veröffentlichungstag der Anmeldung: **16.11.83**
**Patentblatt 83/46**

(84) Benannte Vertragsstaaten: **AT BE CH DE FR GB IT LI LU NL SE**

(71) Anmelder: **Henkel Kommanditgesellschaft auf Aktien, Postfach 1100 Henkelstrasse 67, D-4000 Düsseldorf-Holthausen (DE)**

(72) Erfinder: **Gerhardt, Werner, Dr., Noldeweg 10, D-4010 Hilden (DE)**
Erfinder: **Lehmann, Rudolf, Dr., Fröbelstrasse 4a, D-4040 Neuss (DE)**

(54) **Substituierte 2-(3-Iod-2-propinyloxy)ethanole, ihre Herstellung und ihre Verwendung als antimikrobielle Substanzen.**

(57) Die Erfindung betrifft substituierte 2-(3-Iod-2-propinyl-oxy)-ethanole der allgemeinen Formel (I)

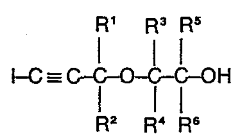

(I)

in der $R^1$ und $R^2$ gleich oder verschieden sind und Wasserstoff, lineare oder verzweigte Alkyl- oder Alkenylreste mit 1 bis 6 Kohlenstoffatomen oder cyclische Alkylreste mit 5 bis 7 Kohlenstoffatomen oder $R^1$ und $R^2$ zusammengenommen $-(CH_2)_n-$ bedeuten, wobei n 4 bis 6 ist,

$R^3$, $R^4$, $R^5$ und $R^6$, die gleich oder verschieden sein können, Wasserstoff, Alkylreste mit 1 bis 4 Kohlenstoffatomen, Arylreste oder $CCl_3$ bedeuten,

mit der Maßgabe, daß wenigstens einer der Reste $R^1$ bis $R^6$ kein Wasserstoff bedeutet, sowie ein Verfahren zur Herstellung dieser Verbindungen und ihre Verwendung als antimikrobielle Substanzen.

EP 0 093 963 A1

4000 Düsseldorf, den 4.5.1982
Henkelstrasse 67

Henkel KGaA
ZR-FE/Patente

Patentanmeldung
D6541 EP

# Substituierte 2-(3-Iod-2-propinyloxy)ethanole, ihre Herstellung und ihre Verwendung als antimikrobielle Substanzen

Die Erfindung betrifft substituierte 2-(3-Iod-2-propinyloxy)ethanole der allgemeinen Formel (I)

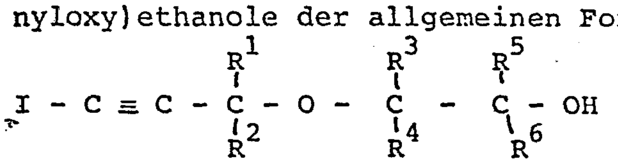

(I)

in der $R^1$ und $R^2$ gleich oder verschieden sind und Wasserstoff, lineare oder verzweigte Alkyl- oder Alkenylreste mit 1 bis 6 Kohlenstoffatomen oder cyclische Alkylreste mit 5 bis 7 Kohlenstoffatomen oder $R^1$ und $R^2$ zusammengenommen $-(CH_2)_n-$bedeuten,, wobei n 4 bis 6 ist,
$R^3$, $R^4$, $R^5$ und $R^6$, die gleich oder verschieden sein können, Wasserstoff, Alkylreste mit 1 bis 4 Kohlenstoffatomen, Arylreste oder $CCl_3$ bedeuten,
mit der Maßgabe, daß wenigstens einer der Reste $R^1$ bis $R^6$ kein Wasserstoff bedeutet.

Die Erfindung betrifft weiterhin ein Verfahren zur Herstellung der substituierten 2-(3-Iod-2-propinyloxy)-ethanole der allgemeinen Formel (I), bei dem in einer ersten Reaktionsstufe Propargylalkohol oder substituierte Propargylalkohole mit Ethylenoxid oder substituierten Epoxiden umgesetzt und in einer zweiten Stufe die erhaltenen Produkte iodiert werden.

Die Erfindung betrifft schließlich die Verwendung der Verbindungen der Formel (I) als antimikrobielle Substanzen.

Beispiele für lineare oder verzweigte Alkylreste oder Alkenylreste mit 1 bis 6 Kohlenstoffatomen, für die $R^1$ und $R^2$ stehen, sind Methyl, Ethyl, Propyl, Isopropyl, n-Butyl, Isobutyl, sec.-Butyl, tert.-Butyl, Pentyl, Hexyl und deren verzweigte Isomere, Vinyl, Allyl, Propenyl, Butenyl, Pentenyl und Hexenyl sowie die entsprechenden Isomeren der genannten Alkenyle mit 4, 5 und 6 Kohlenstoffatomen. Beispiele für cyclische Alkylreste mit 5 bis 7 Kohlenstoffatomen, für die $R^1$ und $R^2$ stehen, sind Cyclopentan, Cyclohexan und Cycloheptan. Bevorzugt werden Verbindungen der Formel I, in denen beide Substituenten $R^1$ und $R^2$ gleichzeitig Wasserstoff oder gleichzeitig Methyl sind, sowie solche, in denen von $R^1$ und $R^2$ ein Substituent Wasserstoff ist während der andere Methyl darstellt.

Beispiele für Alkylreste mit 1 bis 4 Kohlenstoffatomen, für die $R^3$, $R^4$, $R^5$ und $R^6$ stehen, sind Methyl, Ethyl, Propyl, Isopropyl, n-Butyl, Isobutyl, sec.-Butyl und tert.-Butyl,wobei Methyl bevorzugt wird.

Beispiele für Arylreste, für die $R^3$, $R^4$, $R^5$ und $R^6$ stehen, sind Phenyl und Naphthyl.

Bevorzugt werden Verbindungen, in denen $R^1$, $R^2$, $R^3$, $R^4$, $R^5$ und/oder $R^6$ ein niederer Alkylrest mit 1 - 4 Kohlenstoffatomen, insbesondere Methyl oder Ethyl ist. Insbesondere bevorzugt werden die ein- bzw. zweifach methylsubstituierten Derivate.

Die Herstellung der substituierten 2-(3-Iod-2-propinyloxy)ethanole der allgemeinen Formel (I) erfolgt auf die im Folgenden dargelegte Weise.

Die substituierten 2-(3-Iod-2-propinyloxy)-ethanole I, Ia und Ib wurden durch Umsetzung von Propargylalkohol bzw. substituierten Propargylalkoholen IIa, IIb mit Ethylenoxid bzw. substituierten Epoxiden IIIa, IIIb zu den Zwischenstufen IVa, IVb (1.Stufe) und nachfolgende Iodierung (2.Stufe) gemäss dem folgenden allgemeinen Reaktionsschema hergestellt.

Allgemeines Reaktionsschema:

Reihe A ($R^{3-6}$ = H) (Variation von $R^{1-2}$)

Reihe B ($R^{1-2}$ = H) (Variation von $R^{3-6}$)

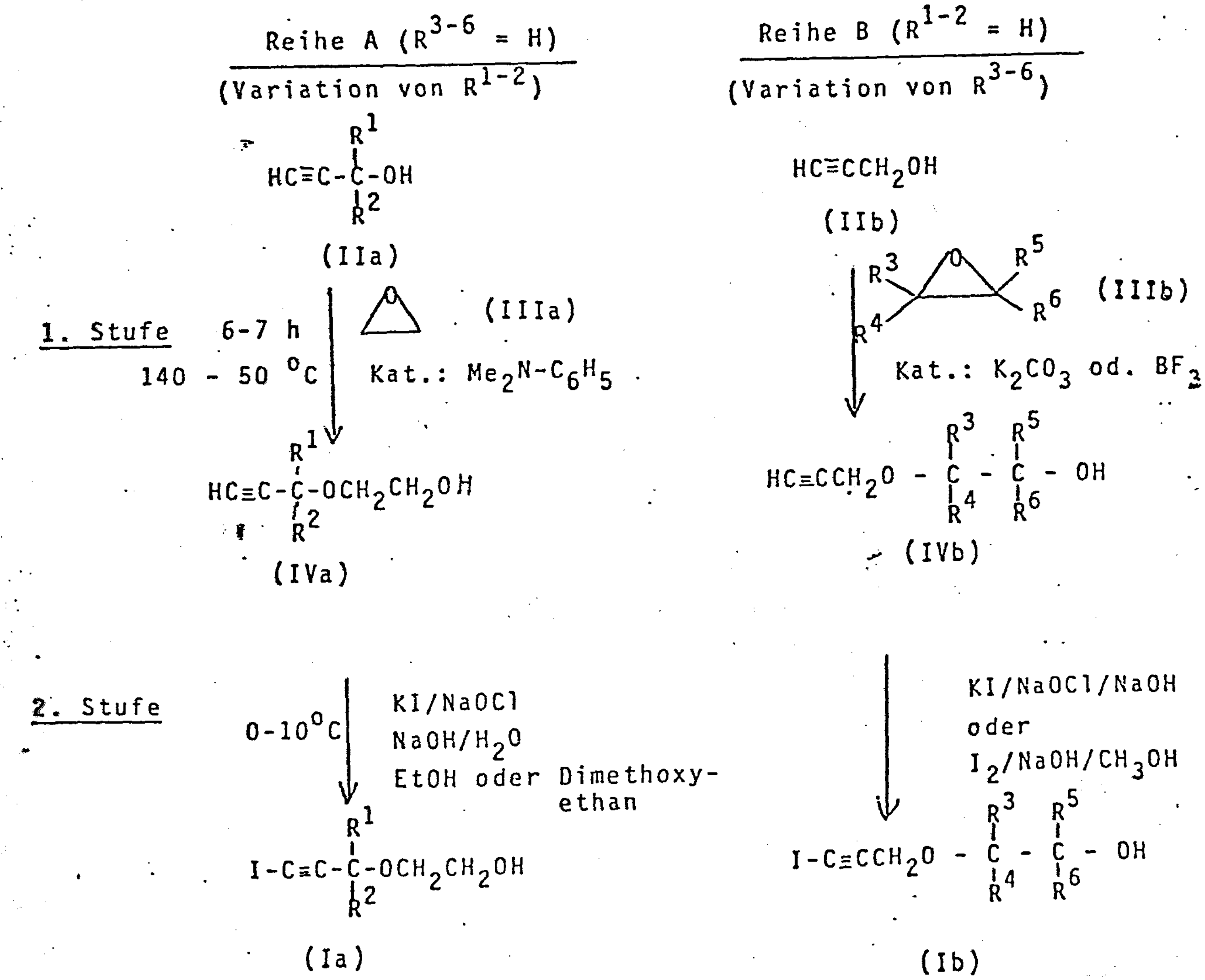


(IIa) (IIb) (IIIa) (IIIb) (IVa) (IVb) (Ia) (Ib)

Wie im Reaktionsschema dargestellt, wurden in der Reihe A die Substituenten $R^1$ und $R^2$ ($R^{3-6}$ = H) und in der Reihe B die Reste $R^{3-6}$ ($R^{1-2}$ = H) variiert.

Es wurden außerdem gleichzeitig die Reste $R^1$, $R^2$ und $R^3$ bis $R^6$ variiert.

Die 1. Reaktionsstufe wird wie folgt durchgeführt: Propargylalkohol bzw. substituierte Propargylalkohole IIa und IIb werden mit Epoxiden IIIa; IIIb zu den entsprechenden substituierten 2-(2-Propinyloxy)-ethanolen IVa, IVb umgesetzt.

Die Synthese der Zwischenprodukte IVa, IVb mit einfachen Alkyl-Substituenten ist in der Literatur bereits beschrieben.

So untersuchten Petrov und Laguneva (J.Gen.Chem. USSR 21, 1361 (1951) die Reaktion von 2-Methyl-3-butin-2-ol (IIa, $R^{1,2}=CH_3$) mit Ethylenoxid IIIa in Gegenwart von N,N-Dimethylanilin als Katalysator zum 2-(2-Methyl-3-butin-2-yloxy)ethanol (IVa, $R^{1,2} = CH_3$).
Alle in Tabelle Ia aufgeführten Zwischenprodukte IVa wurden nach dieser Methode hergestellt.

Die Umsetzungen von unsubstituiertem Propargylalkohol IIb mit verschiedenen Epoxiden IIIb zu den in Tabelle IIa zusammengestellten Zwischenprodukten IVb wurden in Anlehnung an die von Faure und Descotes (Bull.Soc.Chim.-France, 1966, 1569) beschriebenen Reaktionsbedingungen in Gegenwart von basischen ($K_2CO_3$) oder sauren ($BF_3$) Katalysatoren durchgeführt.

Die 2. Reaktionsstufe wird wie folgt durchgeführt: Die substituierten 2-(2-Propinyloxy)ethanole IVa, IVb werden zu den erfindungsgemässen Endprodukten Ia, Ib iodiert.

Die Synthese der 2-(3-Iod-2-propinyloxy)-ethanole mit den allgemeinen Strukturen Ia, Ib ist bisher in der Literatur nicht beschrieben. Die gewünschten Verbindungen wurden nach den folgenden an sich bekannten Iodierungsverfahren 1.) bzw. 2.) hergestellt:

1.) KI/NaOCl/NaOH in Wasser (bei lipophileren Verbindungen in Wasser/Ethanol (1:1) oder in Wasser/Dimethoxyethan).
Hergestellte Verbindungen: Tabelle I und Tabelle II (Verb. I) gemäss Beispielen 1, 2 und 3.

2.) $I_2/NaOH/H_2O$ in Methanol
Hergestellte Verbindungen; Tabelle II (außer Verbindung I) gemäss Beispiel 4.

Die Umsetzung der ersten Reaktionsstufe wird unter Verwendung von Normaldruck bei Temperaturen von 20 bis 100°C, vorzugsweise von 30 bis 80°C durchgeführt. Wenn die Umsetzung im Autoklaven,d.h. unter Druck erfolgt, werden höhere Temperaturen angewandt, z.B. von 140 bis 180°C.

Die Umsetzung der ersten Stufe kann zur Beschleunigung und Verbesserung der Ausbeute mit Hilfe von Katalysatoren durchgeführt werden. Als Katalysatoren haben sich N,N-Dimethylanilin und Kaliumcarbonat als geeignet erwiesen.

Der Fachmann findet in den folgenden Literaturstellen, die sich mit der Umsetzung von Propargylalkohol mit Epoxiden befassen, weitere allgemeine Angaben zu der Durchführung der Umsetzung.

DE-OS 2 241 155, Verfahren zur Herstellung von oxalkylierten alpha-Acetylenalkoholen; G.Dittus in Houben-Weyl, Methoden zur Organischen Chemie, Bd. 6/3, S. 456-7, Thieme Verlag Stuttgart (1965); N. Schönfeldt, Grenzflächenaktive Ethylenoxid-Addukte, S. 17, 32, 84, Wissenschaftliche Verlagsgesellschaft mbH.,Stuttgart 1976 (= Katalysatoren für Ethoxylierungen); DE-AS 1 768 368 (= Propargylalkohol + n EO); A.T. Bottini und J.G. Maroski, J.Org.Chem. 38, 1455 (1973).

Die Umsetzung der zweiten Stufe, d.h. die Iodierung, erfolgt im allgemeinen unter Kühlen bei niedrigen Temperaturen um 0°C beispielsweise zwischen -5 und +10°C, insbesondere zwischen 0 und 5°C.

Die bei der vorliegenden Erfindung zur Anwendung kommenden oben beschriebenen Iodierungsverfahren 1.) und 2.) sind allgemein in der nachfolgend aufgeführten Literatur beschrieben:Houben-Weyl, Methoden der Organischen Chemie, Bd. 5/4 S. 554-7 (1960) und Bd. 5/2a, S. 601, 608 (1977); Methodicum Chimicum, Bd. 7, S. 295 (1976); S.Y. Delavarenne und H.G. Viehe in H.G. Viehe, Chemistry of Acetylenes, Kap. 10, 1-Halogenoacetylenes, S. 685-90, M. Dekker, New York (1969); C. Malen, Bull.-Soc.Chim.France 1957, 904; F. Kai und S. Seki, Chem.Pharm . Bull. Japan, 13, 1374 (1965).

Die erfindungsgemässen Verbindungen eignen sich aufgrund ihrer mikrobistatischen und mikrobiziden Wirkung beispielsweise als Konservierungsmittel für Kosmetika und für die Konservierung technischer Produkte (z.B. Anstrichmittel, Leime, Dispersionen, Kühlschmiermittel), zur antimikrobiellen Ausrüstung von Fugendichtmassen (z.B. Silicondichtmassen), zur Bekämpfung von Schimmelpilzen (wie z.B. Schimmel an feuchten Wänden, Decken oder Fußböden), zum Schützen von Holz sowie zur antimikrobiellen Ausrüstung von Kunststoffen

wie z.B. Polyvinylchlorid (PVC)-Folien. Die erfindungsgemässen Verbindungen können auch zum Desinfizieren verwendet werden.

Zur Verwendung in antimikrobiellen Mitteln können die erfindungsgemässen Verbindungen in bekannter Weise in flüssige, pastenförmige oder feste Zubereitungen eingearbeitet werden wie z.B. wässrige Lösungen, Suspensionen, Emulsionen und Lösungen in organischen Lösungsmitteln.

Die Erfindung wird in den folgenden Bezugsbeispielen, die die Synthese der substituierten 2-(2-Propinyloxy)-ethanole beschreiben und in den Beispielen, die die Synthese der erfindungsgemässen substituierten 2-(3-Iod-2-propinyloxy)ethanole beschreiben, weiter erläutert, wobei die Erfindung jedoch nicht auf die Beispiele beschränkt ist.

<u>Bezugsbeispiel 1</u>

2-(3-Butin-2-yloxy)-ethanol (Verbindung A', IVa, $R^1$ = $CH_3$, $R^2$ = H)

---

Eine Mischung aus 210,3 g (3,0 Mol) 1-Butin-3-ol (IIa, $R^1$ = $CH_3$, $R^2$ = H), 44,0 g (1,0 Mol) Ethylenoxid IIIa und 10 g (0,083 Mol) N,N-Dimethylanilin als Katalysator wurde in einem Autoklaven 7 Stunden bei 148 - 9°C gerührt. Die erhaltene rotbraune Lösung wurde mit 2 l Ether verdünnt und zur Abtrennung des Katalysators mit etherischer HCl-Lösung versetzt (pH ca 4, Ölabscheidung - N,N-Dimethylanilin-Hydrochlorid). Die etherische Lösung wurde anschließend mit 100 ml gesättigter Kochsalz-Lösung gewaschen, über Magnesiumsulfat getrocknet und eingedampft. Bei der fraktionierten Destillation des rotbraunen Öls im Wasserstrahlvakuum über eine Füll-

körperkolonne (Länge: 25 cm) wurden zunächst 145,1 g unumgesetztes 1-Butin-3-ol und nach einigen Zwischenfraktionen 79,0 g (69 % d.Th.) 2-(3-Butin-2-yloxy)-ethanol (IVa, $R^1$=$CH_3$, $R^2$ = H) erhalten (Sdp.$_{21}$ 80°C; $n_D^{20}$ = 1,4447).

$C_6H_{10}O_2$ (114,15)
IR(Film): 3415 (s, OH), 3300 (s, H-C≡C), 2110 (C≡C) $cm^{-1}$
$^1$H-NMR ($CDCl_3$): $\delta$ = 1,45 (d, 3H, $J_1$ = 3,5 Hz, $CH_3$); 2,45 (d, 1H, $J_2$=1Hz, HC=C); 2,6 - 2,9 (breit, OH); 3,4 - 4,0 (m, 4H, $OCH_2CH_2O$); 4,22 (qd, 1H, $J_1$ = 3,5 Hz, $J_2$ = 1 Hz, CH-C=C).

Die übrigen in Tabelle Ia aufgeführten Zwischenprodukte IVa wurden nach der gleichen Methode hergestellt (Autoklav, 6 - 7 h, 140 - 50°C; Verhältnis R-OH : Epoxid = 3 : 1, bei Verbindung D' 2 : 1 und bei Verbindung G' 1 : 1).

Bezugsbeispiel 2
1-(2-Propinyloxy)-2-propanol (Verbindung I'; IVb, $R^5$ = $CH_3$, $R^{3,4,6}$ = H).

Zu einer Suspension von 2 g Kaliumcarbonat in 112,1 g (2,0 Mol) Propargylalkohol IIb wurden bei Raumtemperatur nach und nach 116,2 g (2,0 Mol) Propylenoxid (IIIb, $R^5$ = $CH_3$, $R^{3,4,6}$ = H) getropft. Die Temperatur wurde in 1 Stunde auf 30 - 35°C erhöht. Anschließend wurde die Reaktionsmischung 4 Stunden unter Rückfluss erhitzt (Innentemperatur 50 - 60°C). Nach Abkühlen und Versetzen mit Ether wurde die organische Phase 3 mal mit 100 ml Wasser gewaschen, über Natriumsulfat getrocknet und eingedampft. Das erhaltene gelbliche Öl

(GC : 2 Substanzen) wurde im Wasserstrahlvakuum über eine Vigreuxkolonne (Länge: 20 cm) fraktioniert destilliert. Nach nochmaliger Destillation der Hauptfraktion erhält man 35,7 g (16 % d.Th.) 1-(2-Propinyloxy)-2-propanol (IVb, $R^5 = CH_3$, $R^{3,4,6} = H$) als farbloses Öl (Sdp.$_{27}$ 88°C; $n_D^{20}$ = 1,4454; GC : 1 Substanz).
$C_6H_{10}O_2$ (114,15)
IR (Film): 3430 (s, OH), 3300 (s, H-C≡C), 2115 (C≡C), 1101 (s, $CH_2O$) $cm^{-1}$. $^1$H-NMR ($CDCl_3$): $\delta$ = 1,1 (d, 3H, $J_1$ = 3,1 Hz, $CH_3$); 2,4 (t, 1H, $J_2$ = 1,1 Hz, HC≡C); 2,7 (s, 1H, OH); 3,1 - 4,02 (m, 3H, $CH_2CH$); 4,13 (d, 2H, $J_2$ = 1,1 Hz, C≡C$CH_2$). Die übrigen in Tabelle IIa aufgeführten Zwischenprodukte (J', K' und L') wurden nach derselben Synthesemethode hergestellt ($K_2CO_3$ als Katalysator), jedoch wurde das Verhältnis Propargylalkohol : Epoxid auf 3 : 1 erhöht und die Reaktionstemperatur auf 60 - 80 °C gesteigert (6-20 h). Die Zwischenprodukte M'und N' wurden unter Verwendung von Bortrifluorid als Katalysator synthetisiert (60 - 70°C/3h).

<u>Beispiel 1</u>

2(4-Iod-3-butin-2-yloxy)-ethanol (Verbindung A; Ia, $R^1$ = $CH_3$, $R^2$ = H)

---

Zu einer Lösung von 5,07 g (0,044 Mol) 2-(3-Butin-2-yloxy)-ethanol (erhalten nach Bezugsbeispiel 1), 2,4 g (0,06 Mol) NaOH-Plätzchen und 10,0 g (0,06 Mol) Kaliumiodid in 20 ml Wasser und 20 ml Dimethoxyethan tropfte man bei 0 bis 5°C unter Rühren nach und nach 35,4 g (0,06 Mol) 12,6 %ige Natriumhypochloritlösung (exotherme Reaktion). Nach 3 Stunden Nachrühren bei 5 - 10°C und 16 Stunden bei Raumtemperatur wurde das als zweite, schwerere Phase abgeschiedene Endprodukt durch 3malige Extraktion mit Methylenchlorid abgetrennt. Die organische Phase wurde anschließend 3mal mit wenig Wasser neutral gewaschen, über Magnesiumsulfat getrocknet und

im Vakuum eingeengt (9,1 g dunkelbraunes Öl). Nach Destillation im Kugelrohr bei 40 - 50°C/0,4 Torr erhielt man 7,0 g (66 % d.Th.) 2-(4-Iod-3-butin-2-yloxy)-ethanol (Ia, $R^1 = CH_3$, $R^2 = H$) als hellgelbes Öl ($n_D^{20}$ = 1,5422).

$C_6H_9IO_2$ (240,04)

| Analyse (%) | C | H | I |
|---|---|---|---|
| berechnet | 30,02 | 3,78 | 52,87 |
| gefunden | 30,1 | 3,80 | 53,0 |

IR (Film): 3405 (s, OH), 2175 (I-C≡C) $cm^{-1}$; keine H-C≡C-Banden

$^1H$-NMR ($CDCl_3$): $\delta$ = 1,41 (d, 3H, J=3,5 Hz, $CH_3$); 2,1-2,4 (breit, OH); 3,3 - 3,9 (m, 4H, $OCH_2CH_2$); 4,28 (q, 1H, J=3,5 Hz, C≡C-CH).

Beispiel 2

1-(2-Hydroxyethoxy)-1-iodethinyl-cyclohexan (Verbindung H, Ia, $R^{1,2} = -(CH_2)_5-$)

Zu einer Lösung von 8,4 g (0,05 Mol) 1-(2-Hydroxyethoxy)-- 1-ethinyl-cyclohexan (Verbindung H', IVa, $R^{1,2} = -(CH_2)_5-$) 2,4 g (0,06 Mol) NaOH-Plätzchen und 10,0 g (0,06 Mol) Kaliumiodid in 30 ml Wasser und 30 ml Ethanol tropfte man bei 5 - 10 °C in 0,5 Stunden unter Rühren 32,7 g (0,06 Mol) 13,65%iger Natriumhypochlorit-Lösung (exotherme Reaktion). Nach 3 Stunden Nachrühren bei 5 - 10°C und 16 Stunden bei Raumtemperatur wurde das als zweite, schwerere Phase abgeschiedene Reaktionsprodukt durch zweimalige Extraktion mit Methylenchlorid abgetrennt. Die organische Phase wurde anschließend 3mal mit wenig Wasser neutral gewaschen, über Magnesiumsulfat getrocknet und im Vakuum eingeengt. Man erhielt 13,4 g (91 % d.Th.) 1-(2-Hydroxyethoxy)-1-iodethinyl-cyclohexan (Ia, $R^{1,2} = -(CH_2)_5-$) in Form eines gelblichen Feststoffs vom Schmp. 75 - 83°C.

$C_{10}H_{15}IO_2$ (294,13).

| Analyse, % | C | H | I |
|---|---|---|---|
| berechnet | 40,84 | 5,14 | 43,14 |
| gefunden | 40,2 | 5,06 | 43,1 |

IR (KBr): 3400 (s, OH), 2162 (I-C≡C) $cm^{-1}$, keine H-C≡C-Banden.
$^1H$-NMR ($CDCl_3$): $\delta$ = 1,2 .- 2,3 (m, 11H, $(CH_2)_5$ + OH); 3,5 - 3,8 (m, 4H, $OCH_2CH_2$). Die weiteren in Tabelle I aufgeführten Verbindungen wurden nach der Iodierungsmethode von Beispielen 1 und 2 hergestellt, vorwiegend unter Verwendung von Ethanol als Lösungsvermittler.

Beispiel 3

1-(3-Iod-2-propinyloxy)-2-propanol (Verbindung I, Ib, $R^5$ = $CH_3$, $R^{3,4,6}$ = H)

Zu einer gekühlten Lösung von 11,4 g (0,10 Mol) 1-(2-Propinyloxy)-2-propanol (Verbindung I', IVb, $R^5$ = $CH_3$, $R^{3,4,6}$=H, vgl. Bezugsbeispiel 2), 16,6 g (0,1 Mol) Kaliumiodid und 4,0 g (0,1 Mol) NaOH-Plätzchen in 50 ml Wasser tropfte man bei 0 bis 5°C in 1,5 Stunden unter Rühren 65,4 g (o,12 Mol) 13,65%iger Natriumhypochlorit-Lösung (exotherme Reaktion). Nach 2 Stunden Nachrühren bei 0 bis 5°C wurde die Reaktionsmischung 5 mal mit 100 ml Methylenchlorid extrahiert. Die vereinigten organischen Phasen wurden 3 mal mit 100 ml Wasser neutral gewaschen (davon 2 mal mit Zusatz von wenig Natriumthiosulfat zur Entfärbung), über Natriumsulfat getrocknet und im Vakuum eingeengt. Man erhielt 23,1 g (96 % d.Th.) 1-(3-Iod-2-propinyloxy)-2-propanol (Ib, $R^5$=$CH_3$, $R^{3,4,6}$=H) als bräunliches Öl ($n_D^{20}$ = 1,5413).
$C_6H_9IO_2$ (240,04).

| Analyse, % | C | H | I |
|---|---|---|---|
| berechnet | 30,02 | 3,78 | 52,87 |
| gefunden | 31,2 | 3,96 | 49,0 |

IR (Film): 3415 (s, OH), 2180 (I-C≡C) $cm^{-1}$; keine H-C≡C-Banden.

$^1H$-NMR ($CDCl_3$): $\delta$= 1,15 (d, 3H, J=3,2 Hz, $CH_3$); 2,41 (s, 1H, OH); 3,1 - 4,1 (m, 3H, $CH_2CH$); 4,3 (s, 2H, $C{\equiv}CCH_2$).

Die übrigen in Tabelle II aufgeführten Verbindungen wurden nach der in Beispiel 4 beschriebenen Iodierungsmethode hergestellt ($I_2$, NaOH, $CH_3OH$).

Beispiel 4

2-(3-Iod-2-propinyloxy)-cyclohexanol (Verbindung M, Ib, $R^{3,5}$ = $-(CH_2)_4-$, $R^{4,6}$ = H

Zu einer Lösung von 6,2 g (0,04 Mol) 2-(2-Propinyloxy)-cyclohexanol (Verbindung M', IVb, $R^{3,5}$ = $-(CH_2)_4-$, $R^{4,6}$ = H) in 100 ml Methanol tropfte man unter Kühlen ca. ein Drittel einer Lösung von 6,0 g (0,15 Mol) NaOH-Plätzchen in 20 ml Wasser. Dazu gab man in 1 Stunde bei Raumtemperatur portionsweise 11,2 g (0,044 Mol) Iod und die Restmenge der NaOH-Lösung. Nach 3 Stunden Nachrühren bei 20 - 25°C versetzte man das Reaktionsgemisch mit 100 ml Wasser und extrahierte 3 mal mit Ether. Die vereinigten organischen Phasen wurden 3 mal mit Wasser gewaschen, über Magnesiumsulfat getrocknet und im Vakuum eingeengt. Man erhielt 10,2 g (91% d.Th.) 2-(3-Iod-2-propinyloxy)-cyclohexanol (Ib, $R^{3,5}$ = $-(CH_2)_4-$, $R^{4,6}$ = H) als viskoses Öl, das zu einer gelblichen Festsubstanz vom Schmp. 45 - 55°C erstarrte.

$C_9H_{13}IO_2$ (280,10)

| Analyse, % | C | H | I |
|---|---|---|---|
| berechnet | 38,59 | 4,68 | 45,31 |
| gefunden | 38,9 | 4,63 | 44,9 |

IR (Schmelze): 3440 (OH), 2185 (I-C≡C) $cm^{-1}$; keine H-C≡C-Banden.
$^1H$-NMR ($CDCl_3$): $\delta$ = 0,95 - 2,22 (m, breit, 8H, $CH_2$); 2,6 (breit, OH); 2,95 - 3,7 (m, breit, 2H, HOCH-CH-O); 4,38 (s, 2H, C≡$CCH_2$).

Tabelle I

Physikalische Daten von Verbindungen gem. der Erfindung

| Verbindung | Chem. Struktur $I-C\equiv C-C(R^1)(R^2)-OCH_2CH_2OH$ $R^1$ | $R^2$ | Schmp (°C) oder $n_D^{20}$ | Ausbeute (%) |
|---|---|---|---|---|
| A | H | $CH_3$ | gelbl. Öl 1,5422 | 66 |
| B | $CH_3$ | $CH_3$ | 1,5213 | 72 |
| C | $CH_3$ | $CH_2CH_3$ | Kristalle, weich Schmp. >35° | 64 |
| D | $CH_3$ | $CH_2CH_2CH_3$ | 1,5212 | 86 |
| E | $CH_3$ | $CH(CH_3)_2$ | Kristallbrei schwarz | 75 |
| F | $CH_3$ | $CH_2CH_2CH=C(CH_3)_2$ | Harz, schwarz | 79 |
| G | $CH_2CH_3$ | $CH_2CH_3$ | Kristallbrei schwarz | 76 |
| H | $-(CH_2)_5-$ | | Feststoff, gelbl., 75 - 83°C | 91 |

Tabelle I a

Physikalische Daten von Ausgangsverbindungen

| Verb. | Chem. Struktur $HC\equiv C-C(R^1)(R^2)-OCH_2CH_2OH$ $R^1$ | $R^2$ | Sdp.(°C/mbar) oder $n_D^{20}$ | Ausbeute (%) |
|---|---|---|---|---|
| A' | H | $CH_3$ | 80/21<br>1,4447 | 69 |
| B' | $CH_3$ | $CH_3$ | 85-6/28<br>1,4432 | 33 |
| C' | $CH_3$ | $CH_2CH_3$ | 83-8/22<br>1,4520 | 7 |
| D' | $CH_3$ | $CH_2CH_2CH_3$ | 99-102/21<br>1,4484 | 14 |
| E' | $CH_3$ | $CH(CH_3)_2$ | 97-105/20<br>1,4607 | 10 |
| F' | $CH_3$ | $CH_2CH_2CH=C(CH_3)_2$ | 85-92/0,4<br>1,4712 | 8 |
| G' | $CH_2CH_3$ | $CH_2CH_3$ | 93/19<br>1,4612 | 3 |
| H' | $-(CH_2)_5-$ | | 78-82/0,4<br>1,4831 | 32 |

Tabelle II

Physikalische Daten von Verbindungen gemäss der Erfindung

| Verb. | Chem. Struktur $I\text{-}C{\equiv}CCH_2O\overset{R^3}{C}H — \overset{R^5}{C}H — OH$ | | Schmp. (°C) oder $n_D^{20}$ | Ausbeute (%) |
|---|---|---|---|---|
| | $R^3$ | $R^5$ | | |
| I | H | $CH_3$ | 1,5413 | 96 |
| J | H | $C_6H_5$ | 1,5906 | 63 |
| K | H | $CCl_3$ | 1,5642 | 75 |
| L | $CH_3$ | $CH_3$ | 1,5336 | 80 |
| M | $-(CH_2)_4-$ | | 45 - 55°C | 91 |
| N | $I\text{-}C{\equiv}C\text{-}\overset{CH_3}{C}H\text{-}O\text{-}\overset{CH_3}{C}H\text{-}\overset{CH_3}{C}H\text{-}OH$ | | 1,5181 | 76 |

Tabelle II a

Physikalische Daten von Ausgangsverbindungen

| Verbindung | Chem. Struktur $HC{\equiv}CCH_2OCH(R^3)-CH(R^5)-OH$ | | Sdp. (°C/mbar) $n_D^{20}$ | Ausbeute |
|---|---|---|---|---|
| | $R^3$ | $R^5$ | | |
| I' | H | $CH_3$ | 88/27<br>1,4454 | 16 |
| J' | H | $C_6H_5$ | 109/0,15<br>1,5334 | 63 |
| K' | H | $CCl_3$ | 86/0,2<br>1,5038 | 22 |
| L' | $CH_3$ | $CH_3$ | 84/22<br>1,4473 | 13 |
| M' | $-(CH_2)_4$ | | 83/0,2<br>1,4847 | 17 |
| N' | $H-C{\equiv}C-CH(CH_3)-O-CH(CH_3)-CH(CH_3)-OH$ | | 1,4399 | 56 |

Antimikrobielle Wirksamkeit der Verbindungen der allgemeinen Formel (I)

Die mikrobistatische Wirksamkeit der Verbindungen A bis N wurde gegenüber folgenden Testkeimsuspensionen bestimmt:

| | |
|---|---|
| 1. Staphylococcus aureus | $2 \times 10^9$ Keime/ml |
| 2. Escherichia coli | $2 \times 10^9$ Keime/ml |
| 3. Pseudomonas aeruginosa | $5 \times 10^8$ Keime/ml |
| 4. Candida albicans | $2 \times 10^8$ Keime/ml |
| 5. Aspergillus niger | $5 \times 10^7$ Keime/ml |
| 6. Penicillium camerunense | $5 \times 10^7$ Keime/ml |
| 7. Trichophyton mentagrophytes | $2 \times 10^7$ Keime/ml |

Die Hemmkonzentrationen der zu untersuchenden Verbindungen wurden mit Hilfe des Verdünnungstests nach den Richtlinien für die Prüfung chemischer Desinfektionsmittel der Deutschen Gesellschaft für Hygiene und Mikrobiologie (1972) ermittelt. Die Versuche wurden in sterilen Reagenzröhrchen ausgeführt, die Standard-I-Bouillon (pH 7,5 Merck) oder Würze-Bouillon ( pH 5,5, Merck, 8° BG) enthielten. Nach Zugabe der Wirkstoffe betrug das Nährlösungsvolumen in den Röhrchen jeweils 10 ml. Anschließend wurden jeweils 0,1 ml der Testkeimsuspension der angegebenen Konzentration in die Röhrchen gebracht. Die mit Bakterien beimpften Nährlösungsproben wurden 3 Tage lang bei 37°C im Brutschrank aufbewahrt. Die mit Pilzen beimpften Proben wurden 3 bis 7 Tage lang bei 30°C bebrütet. Danach wurde festgestellt, welche dem Nährmedium zugeführte Wirkstoffkonzentration das Wachstum der Keime gerade noch gehemmt hatte. Der auf diese Weise gefundene Wert wurde als Hemmkonzentration bezeichnet. Folgende Wirkstoffkonzentrationen in ppm wurden getestet: 1 000, 500, 250, 100, 50,25, 10, 5, 2,5.

Tabelle III

Flüssige Hemmreihe zum Nachweis der mikrobistatischen Wirkung (X = > 1000 ppm)

| Verbindung | Testkeim 1 | 2 | 3 | 4 | 5 | 6 | 7 |
|---|---|---|---|---|---|---|---|
| A | 100 | 100 | 500 | 100 | 50 | 50 | - |
| B | 10 | 100 | 1000 | 100 | 50 | 50 | 25 |
| C | 100 | 500 | 1000 | 250 | 25 | 50 | 25 |
| D | 50 | 500 | 1000 | 100 | 25 | 25 | 25 |
| E | 50 | 1000 | X | 100 | 50 | 50 | 50 |
| F | 50 | X | X | 50 | ≤10 | 50 | - |
| G | 50 | 1000 | X | 100 | 50 | 50 | 25 |
| H | 100 | 1000 | 1000 | 100 | 50 | 50 | - |
| I | 100 | 250 | 1000 | 25 | 5 | 10 | 5 |
| J | 100 | 500 | X | 50 | 2,5 | 5 | 5 |
| K | 50 | 250 | 1000 | 50 | ≤10 | ≤10 | - |
| L | 100 | 500 | X | 50 | 2,5 | 10 | 25 |
| M | 50 | 500 | X | 25 | 5 | 25 | 5 |
| N | 100 | 1000 | X | 100 | ≤10 | 50 | - |

Die mikrobizide Wirkung der Verbindungen A bis H wurde gegenüber folgenden Testkeimsuspensionen bestimmt:

1. Aspergillus niger $5 \times 10^7$ Keime/ml
2. Penicillium camerunense $5 \times 10^7$ Keime/ml

Die Abtötungszeiten der zu untersuchenden Produkte wurden mit Hilfe des Suspensionstests nach den Richtlinien für die Prüfung chemischer Desinfektionsmittel der Deutschen Gesellschaft für Hygiene und Mikrobiologie (1972) ermittelt. Die zu prüfenden Substanzen wurden zunächst in wenig Alkohol gelöst. Aus den ethanolischen Lösungen wurde durch Verdünnen mit Hartwasser einer Härte von 17° dH Testlösungen hergestellt, die 3000 ppm und 500 ppm Wirkstoff und maximal 1 Gew.-% Ethanol enthielten.

Es wurden bei Raumtemperatur jeweils 0,1 ml der Testkeimsuspension in Reagenzgläser pipettiert. Hierzu wurden jeweils 10 ml der oben beschriebenen Testlösung gegeben. Nach Einwirkungszeiten von 15, 60 und 120 Minuten bei Raumtemperatur wurde den Reagenzgläsern mit Hilfe einer Öse ein Tropfen Material entnommen und in 10 ml Nährlösung, die 3 % Tween 80 und 0,3 % Lecithin als Enthemmer enthielt, überimpft. Das Nährmedium bestand aus 1 Gew.-%iger Standard-I-Bouillion (Merck). Die Proben wurden bei 30°C bebrütet. Nach frühestens 5 Tagen wurden die Kulturen makroskopisch auf Wachstum beurteilt und auf diesem Weg die Abtötungszeiten ermittelt.

Die Ergebnisse sind in der folgenden Tabelle IV aufgeführt.

Tabelle IV

Mikrobizide Wirkung im Suspensionstest (X = > 120min)

| Verbindung | Abtöt. konz. (ppm) | Asp. niger | Pen. camer. | pH |
|---|---|---|---|---|
| A | 3000 | ≤15 | ≤15 | 6,0 |
| | 500 | X | ≤15 | |
| B | 3000 | ≤15 | ≤15 | 4,7 |
| | 500 | X | X | |
| C | 3000 | 60 | ≤15 | 6,7 |
| | 500 | X | X | |
| D | 3000 | X | X | 4,2 |
| E | 3000 | X | X | 4,2 |
| F | 3000 | X | X | 4,4 |
| G | 3000 | X | X | 4,4 |
| H | 3000 | X | X | 4,4 |
| I | 3000 | ≤15 | ≤15 | 4,6 |
| | 500 | X | ≤15 | |
| J | 3000 | 60 | ≤15 | 4,9 |
| | 500 | 120 | ≤15 | |
| K | 3000 | 60 | ≤15 | 3,9 |
| | 500 | X | X | |
| L | 3000 | ≤15 | ≤15 | 4,4 |
| | 500 | ≤15 | ≤15 | |
| M | 3000 | ≤15 | ≤15 | 4,9 |
| | 500 | ≤15 | ≤15 | |
| N | 3000 | ≤15 | ≤15 | 4,2 |
| | 500 | X | ≤15 | 4,6 |

Patentansprüche

1. Substituierte 2-(3-Iod-2-propinyloxy)-ethanole der allgemeinen Formel (I)

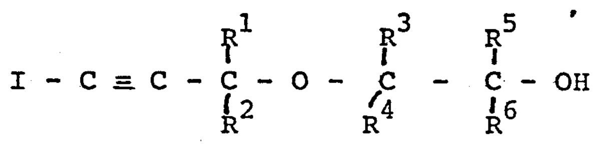

(I)

in der $R^1$ und $R^2$ gleich oder verschieden sind und Wasserstoff, lineare oder verzweigte Alkyl-oder Alkenylreste mit 1 bis 6 Kohlenstoffatomen oder cyclische Alkylreste mit 5 bis 7 Kohlenstoffatomen oder $R^1$ und $R^2$ zusammengenommen $-(CH_2)_n-$ bedeuten, wobei n 4 bis 6 ist,
$R^3$, $R^4$, $R^5$ und $R^6$, die gleich oder verschieden sein können, Wasserstoff, Alkylreste mit 1 bis 4 Kohlenstoffatomen, Arylreste oder $CCl_3$ bedeuten, mit der Maßgabe, daß wenigstens einer der Reste $R^1$ bis $R^6$ kein Wasserstoff bedeutet.

2. Verfahren zur Herstellung von Verbindungen nach Anspruch 1, dadurch gekennzeichnet, daß man in einer ersten Reaktionsstufe Propargylalkohol und/oder substituierte Propargylalkohole der allgemeinen Formeln IIa und IIb

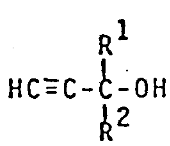

(IIa)

$HC\equiv CCH_2OH$

(IIb)

mit Ethylenoxid und/oder substituierten Epoxiden der allgemeinen Formeln IIIa und IIIb

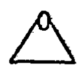 (IIIa)

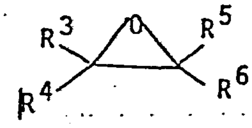 (IIIb)

umsetzt und in einer zweiten Reaktionsstufe die erhaltenen Zwischenprodukte iodiert, wobei $R^1$, $R^2$, $R^3$, $R^4$, $R^5$ und $R^6$, die im Anspruch 1 angegebenen Bedeutungen haben.

3. Antimikrobielle Mittel, enthaltend wenigstens eine Verbindung nach Anspruch 1 zusammen mit üblichen Formulierungsstoffen.

4. Verwendung der Verbindungen nach Anspruch 1 als antimikrobielle Substanzen.

Patentansprüche für Österreich

1. Verfahren zur Herstellung von substituierten 2-(3-Iod-2-propinyloxy)-ethanolen der allgemeinen Formel (I)

$$\begin{array}{ccccccccccccc} & & & & R^1 & & & & R^3 & & R^5 & & \\ & & & & | & & & & | & & | & & \\ I & - & C \equiv C & - & C & - & O & - & C & - & C & - & OH \\ & & & & | & & & & | & & | & & \\ & & & & R^2 & & & & R^4 & & R^6 & & \end{array} \qquad (I)$$

in der $R^1$ und $R^2$ gleich oder verschieden sind und Wasserstoff, lineare oder verzweigte Alkyl- oder Alkylenreste mit 1 bis 6 Kohlenstoffatomen oder cyclische Alkylreste mit 5 bis 7 Kohlenstoffatomen oder $R^1$ und $R^2$ zusammengenommen $-(CH_2)_n-$ bedeuten, wobei n 4 bis 6 ist, $R^3$, $R^4$, $R^5$ und $R^6$, die gleich oder verschieden sein können, Wasserstoff, Alkylreste mit 1 bis 4 Kohlenstoffatomen, Arylreste oder $CCl_3$ bedeuten, mit der Maßgabe, daß wenigstens einer der Reste $R^1$ bis $R^6$ kein Wasserstoff bedeutet, dadurch gekennzeichnet, daß man in einer ersten Reaktionsstufe Propargylalkohol und/oder substituierte Propargylalkohole der allgemeinen Formeln IIa und IIb

$$\begin{array}{ccc} & & R^1 \\ & & | \\ HC \equiv C & - & C - OH \\ & & | \\ & & R^2 \end{array}$$

(IIa)

$$HC \equiv CCH_2OH$$

(IIb)

mit Ethylenoxid und/oder substituierten Epoxiden der allgemeinen Formeln IIIa und IIIb

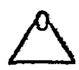 (IIIa)

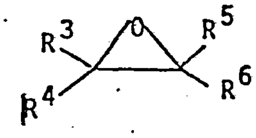 (IIIb)

umsetzt und in einer zweiten Reaktionsstufe die erhaltenen Zwischenprodukte iodiert, wobei $R^1$, $R^2$, $R^3$, $R^4$, $R^5$ und $R^6$, die die für die Formel I angegebenen Bedeutungen haben.

2. Antimikrobielle Mittel, enthaltend wenigstens eine Verbindung der Formel I in Anspruch 1 zusammen mit üblichen Formulierungsstoffen.

3. Verwendung der Verbindungen der Formel I in Anspruch 1 als antimikrobielle Substanzen.

0093963

Europäisches Patentamt

# EUROPÄISCHER RECHERCHENBERICHT

Nummer der Anmeldung

EP 83 10 4152

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | CHEMICAL ABSTRACTS, Band 83, Nr. 13, 29. September 1975, Seite 492, Nr. 113679k, Columbus, Ohio, USA & JP - A - 75 09765 (MEIJI CONFECTIONARY CO., LTD.) 16.04.1975 --- | 1,3,4 | C 07 C 43/178<br>C 07 C 43/196<br>C 07 C 41/03<br>C 07 C 41/22<br>A 01 N 31/00 |
| A | GB-A-1 139 455 (HOFFMANN-LA ROCHE) * Seite 1; Seite 2, Zeilen 1-23; Beispiel 9 * ----- | 1-4 | |
| | | | **RECHERCHIERTE SACHGEBIETE (Int. Cl. 3)** |
| | | | C 07 C 43/00 |

Der vorliegende Recherchenbericht wurde für alle Patentansprüche erstellt.

| Recherchenort | Abschlußdatum der Recherche | Prüfer |
|---|---|---|
| DEN HAAG | 20-06-1983 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82